# EUROPEAN PATENT APPLICATION

(11) **EP 4 559 541 A1**
(43) Date of publication of application: **28.05.2025**
(21) Application number: 23886261.9
(22) Date of filing: 01.11.2023
(51) Int. Cl.: A63B 71/06, A63B 24/00, G04G 21/02

(54) **SYSTEM AND METHOD FOR GENERATING WORKOUT ROUTINE**

(30) Priority: 02.11.2022 KR 20220144351; 10.11.2022 KR 20220149278
(71) Applicant: Samsung Electronics Co., Ltd., Suwon-si, Gyeonggi-do 16677 (KR)
(72) Inventor: KIM, Kyoungae, Suwon-si Gyeonggi-do 16677 (KR); KIM, Hyeonseong, Suwon-si Gyeonggi-do 16677 (KR); PARK, Sunmi, Suwon-si Gyeonggi-do 16677 (KR); BAEK, Kumhwa, Suwon-si Gyeonggi-do 16677 (KR)
(74) Representative: Nederlandsch Octrooibureau
(86) International application number: PCT/KR2023/017243
(87) International publication number: WO 2024/096565

(57) **Abstract**

A system for generating a workout routine according to an embodiment may include a wearable electronic device that collects workout data corresponding to a plurality of workout activities performed by a user wearing the wearable device on the basis of the user's biological data acquired from a sensor module of the wearable device, and transmits the collected workout data to an electronic device. The system according to an embodiment may include the electronic device that generates the workout routine of the user on the basis of the workout data received from the wearable electronic device and recommends changing at least one of the types of workout included in the workout routine, a workout duration, the number of workouts, a rest duration, or the number of rests according to body information about the user.

## Description

### [Technical Field]

The disclosure relates to a system and method for generating a workout routine.

### [Background Art]

Wearable electronic devices may provide a function capable of monitoring biometric information at home rather than in a hospital, as semiconductor integration and sensing technology advances. Accordingly, the number of users exercising with wearable electronic devices capable of monitoring biometric information is increasing.

If a user wearing a wearable electronic device selects a type of exercise on the wearable electronic device, performs and then ends the exercise, the wearable electronic device may provide exercise information, such as the user's heart rate, calories burned, and/or exercise distance, while performing the exercise according to the selected type of exercise.

### [Detailed Description of the Invention]

### [Technical Solution]

A system for generating an exercise routine, according to an embodiment, may comprise a wearable electronic device configured to collect exercise data corresponding to a plurality of exercise actions performed by a user wearing the wearable electronic device based on biometric information of the user obtained from a sensor module of the wearable electronic device and transmit the collected exercise data to an electronic device.

The system according to an embodiment may comprise an electronic device configured to generate the user's exercise routine based on the exercise data received from the wearable electronic device and recommend a change of at least one of a type of exercise, an exercise time, a number of times of exercise, a rest time, or a number of times of rest included in the exercise routine according to the user's body information.

A method for generating an exercise routine, according to an embodiment, may comprise collecting, by a wearable electronic device, exercise data corresponding to a plurality of exercise actions performed by a user wearing the wearable electronic device based on biometric information of the user obtained from a sensor module of the wearable electronic device and transmitting the collected exercise data to an electronic device.

The method according to an embodiment may comprise generating the user's exercise routine based on the exercise data received from the wearable electronic device, by the electronic device.

The method according to an embodiment may comprise recommending, by the electronic device, the change of at least one of the type of exercise, the exercise time, the number of times of exercise, the rest time, or the number of times of rest included in the exercise routine based on the user's body information while visually displaying the exercise routine on a display of the electronic device.

### [Brief Description of the Drawings]

FIG. 1 is a view illustrating an electronic device in a network environment according to an embodiment;
FIG. 2 is a view illustrating a system for managing a battery of a wearable electronic device according to an embodiment;
FIG. 3 is a block diagram illustrating a wearable electronic device according to an embodiment;
FIG. 4 is a block diagram illustrating an electronic device according to an embodiment;
FIG. 5 is a view illustrating an operation of detecting exercise start on a wearable electronic device according to an embodiment;
FIG. 6 is a view illustrating an exercise action and a rest action on a wearable electronic device according to an embodiment;
FIG. 7 is a view illustrating an operation of classifying an exercise type based on exercise data on an electronic device according to an embodiment;
FIGS. 8A, 8B, and 8C are views illustrating an operation of generating an exercise routine and recommending an exercise routine based on user's body information on an electronic device according to an embodiment;
FIGS. 9A, 9B, 9C, and 9D are views illustrating an operation of editing an exercise routine according to an embodiment;
FIG. 10 is a view illustrating an operation of providing a guide while executing an exercise routine on a wearable electronic device according to an embodiment;
FIG. 11 is a flowchart illustrating an operation for generating an exercise routine according to an embodiment;
FIG. 12 is a flowchart illustrating an operation for generating an exercise routine on a wearable electronic device according to an embodiment;
FIG. 13 is a flowchart illustrating an operation for generating an exercise routine on an electronic device according to an embodiment; and
FIG. 14 is a flowchart illustrating an operation for classifying an exercise type on an electronic device according to an embodiment.

### [Mode for Carrying out the Invention]

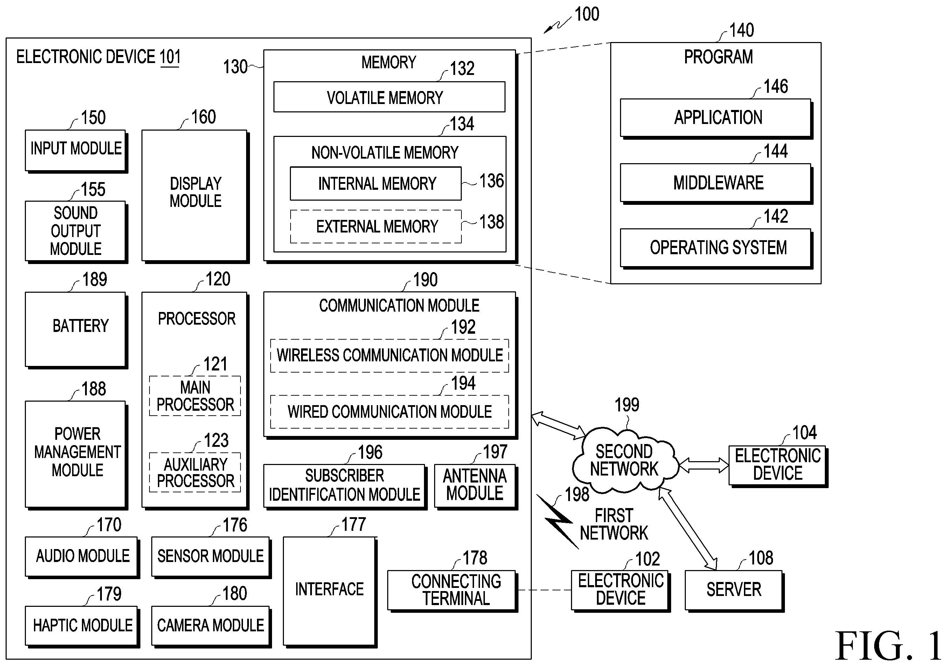

FIG. 1 is a block diagram illustrating an electronic device 101 in a network environment 100 according to an embodiment. Referring to FIG. 1, the electronic device 101 in the network environment 100 may communicate with at least one of an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In an embodiment, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display 160 may include, for example, a display, a hologram device, or a projector and control circuitry to control a corresponding one of the display, hologram device, and projector. According to an embodiment, the display 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to an embodiment, the antenna module 197 may form a mmWave antenna module. According to an embodiment, the mm Wave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, instructions or data may be transmitted or received between the electronic device 101 and the external electronic device 104 via the server 108 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. **In** another embodiment, the external electronic device 104 may include an Internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

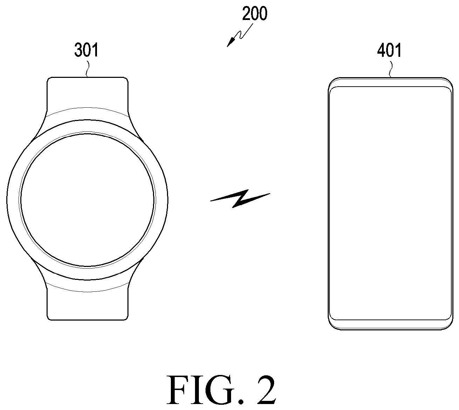

FIG. 2 is a view 200 illustrating a system for generating an exercise routine according to an embodiment.

Referring to FIG. 2, a system for generating an exercise routine may include a wearable electronic device 301 and an electronic device 401.

The exercise routine may indicate an exercise sequence and/or an exercise method in which the user may regularly perform exercise. As the exercise routine according to an embodiment includes at least one of the type of exercise, exercise time, number of times of exercise, rest time, or number of times of rest, the exercise routine may provide the user with a regular exercise sequence and/or exercise method, as well as a rest time and number of times of rest for the user performing the exercise.

According to an embodiment, the wearable electronic device 301 may collect exercise data corresponding to a plurality of exercise actions performed by the user wearing the wearable electronic device based on biometric information obtained by a sensor module of the wearable electronic device, and transmit the collected exercise data to the electronic device 401.

According to an embodiment, the wearable electronic device 301 may detect the user's exercise start and exercise end, and transmit the collected exercise data from the exercise start to the exercise end to the electronic device 401.

According to an embodiment, the wearable electronic device 301 may detect the user's exercise start based on at least one of reception of the user's selection indicating an exercise start, identification of an exercise schedule stored in the wearable electronic device, detection of the wearable electronic device being positioned in an exercise-related place, or detection of the user repeatedly performing the same action.

According to an embodiment, if the wearable electronic device 301 detects the user's rest action between the plurality of exercise actions while collecting the exercise data, it may collect rest data corresponding to the rest action and transmit the collected rest data to the electronic device 401. When exercise data corresponding to the user's first exercise action is not collected within a first predetermined time (e.g., 30 minutes) while collecting exercise data corresponding to the user's first exercise action, and if the user's heart rate detected while performing the first exercise action decreases below a threshold, the wearable electronic device 301 may detect the user's rest action and transmit rest data including the rest time and the number of times of rest performed to the electronic device 401.

According to an embodiment, the wearable electronic device 301 may detect the end of the exercise if the biometric information is not obtained even after the first predetermined time (e.g., 30 minutes) for detecting the rest action while collecting the exercise data from the time when the exercise start is detected.

According to an embodiment, the wearable electronic device 301 may receive an exercise routine generated based on exercise data corresponding to the plurality of exercise actions from the electronic device, and may provide the exercise routine through a display or speaker of the wearable electronic device 301.

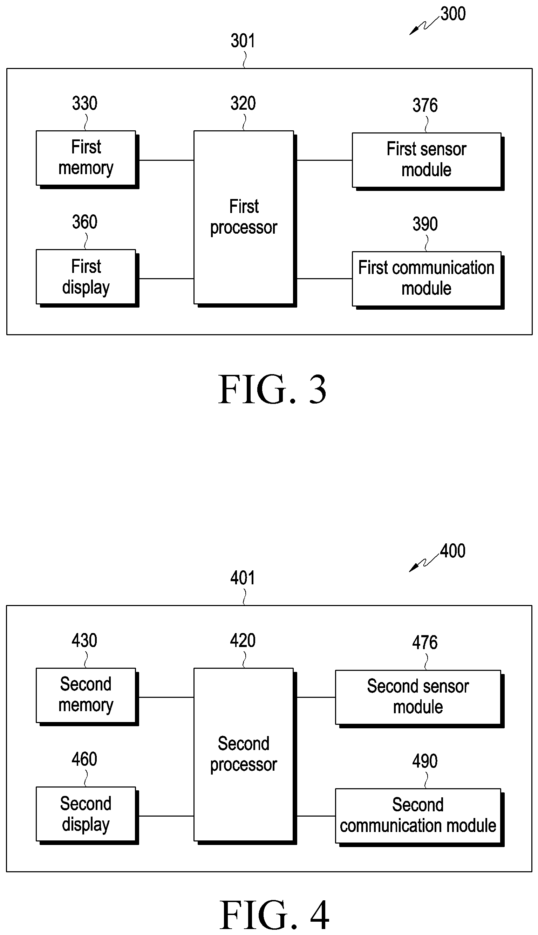

The configuration of the wearable electronic device 301 may be described in detail in FIG. 3.

According to an embodiment, the electronic device 401 may generate the user's exercise routine if receiving exercise data corresponding to the plurality of exercise actions performed by the user wearing the wearable electronic device from the wearable electronic device 301.

According to an embodiment, the electronic device 401 may detect the type of exercise, exercise time, and number of times of exercise based on the exercise data received from the wearable electronic device 301, and generate the exercise routine of the user based on the detected type of exercise, exercise time, and number of times of exercise.

According to an embodiment, the electronic device 401 may detect a periodic repetitive exercise pattern based on the exercise data received from the wearable electronic device 301, perform grouping based on the periodic repetitive exercise pattern, and classify the type of exercise per grouping using training data.

According to an embodiment, the electronic device 401 may detect the rest time and the number of times of rest when the user has rested between the plurality of exercise actions performed by the user based on the rest data received from the wearable electronic device 301, and may generate the exercise routine by including the detected rest time and number of times of rest in the exercise routine.

According to an embodiment, if the user's exercise routine is generated, the electronic device 401 may recommend a change of at least one of the type of exercise, exercise time, number of times of exercise, rest time, or number of times of rest included in the exercise routine according to the user's body information.

According to an embodiment, the electronic device 401 may provide an edit function in which at least one of the type of exercise, the exercise time, the number of exercises, the rest time, or the number of times of rest included in the exercise routine may be changed by the user.

According to an embodiment, the electronic device 401 may transmit the exercise routine to the wearable electronic device 301 so that the exercise routine may be provided from the wearable electronic device 301.

The configuration of the electronic device 401 may be described in detail in FIG. 4.

FIG. 3 is a block diagram 300 illustrating a wearable electronic device according to an embodiment.

Referring to FIG. 3, according to an embodiment, a wearable electronic device 301 (e.g., the wearable electronic device 301 of FIG. 2) may include a first processor 320, first memory 330, a first display 360, a first sensor module 376, and a first communication module 390.

At least some of the components of the electronic device 301 illustrated in FIG. 3 may be identical or similar to the components of the electronic device 101 of FIG. 1, and redundant descriptions will be omitted below.

According to an embodiment, the first processor 320 may be implemented to be substantially identical or similar to the processor 120 of FIG. 1.

According to an embodiment, the first processor 320 may collect exercise data corresponding to a plurality of exercise actions performed by the user wearing the wearable electronic device 301 based on the biometric information obtained through the first sensor module 376, and transmit the collected exercise data to an electronic device (e.g., the electronic device 401 of FIG. 3) communicatively connected through the first communication module 390.

According to an embodiment, if the user's exercise start is detected, the first processor 320 may transmit information indicating the user's exercise start to the electronic device.

According to an embodiment, if receiving the user's selection indicating the start of the exercise, the first processor 320 may detect the user's exercise start. For example, the first processor 320 may detect the user's exercise start if receiving a selection of one exercise type among a plurality of exercise types provided by the wearable electronic device or the user's selection requesting to generate an exercise routine.

According to an embodiment, if identifying an exercise schedule stored in the wearable electronic device or the electronic device, the first processor 320 may detect the user's exercise start. For example, the first processor 320 may detect an exercise schedule in a calendar application, a notification application, and/or a message application, store the detected exercise schedule, and detect the start time of the exercise schedule as the user's exercise start time.

According to an embodiment, the first processor 320 may identify the exercise schedule stored in the wearable electronic device and detect the user's exercise start if the exercise data is collected. For example, the first processor 320 is a calendar application. The exercise schedule may be detected in the notification application and/or the message application, the exercise schedule may be stored, and if the exercise data is collected at the start time of the detected exercise schedule, it may be detected as the user's exercise start time.

According to an embodiment, if the wearable electronic device 301 is located in an exercise-related place, the first processor 320 may detect the user's exercise start. For example, if the wearable electronic device 301 is located in a place where the user's exercise information is recorded or where the exercise facility is located, the user's exercise start may be detected.

According to an embodiment, if the wearable electronic device 301 is located in the exercise-related place and the exercise data is collected, the first processor 320 may detect the user's exercise start. For example, if the exercise data is collected while the wearable electronic device 301 is located in the place where the user's exercise information is recorded or where the exercise facility is located, the user's exercise start may be detected.

According to an embodiment, if the user's exercise start is detected, the first processor 320 may collect exercise data based on biometric information obtained through the first sensor module 376, and transmit the collected exercise data to the electronic device.

According to an embodiment, if the user's rest action is detected while collecting the exercise data and transmitting the collected exercise data to the electronic device, the first processor 320 may transmit rest data corresponding to the rest action to the electronic device.

According to an embodiment, if the exercise data corresponding to the user's first exercise action is not collected within the first predetermined time (e.g., 30 minutes) while collecting the exercise data corresponding to the first exercise action among the plurality of exercise actions performed by the user, and the user's heart rate detected while performing the first exercise action decreases below a threshold, the first processor 320 may detect the user's rest action and transmit rest data including the rest time and the number of times of rest when the rest action has been performed to the electronic device.

According to an embodiment, if the exercise data is not collected even after the first predetermined time for detecting the user's rest action elapses while collecting the exercise data and transmitting the collected exercise data to the electronic device, the first processor 320 may detect the user's exercise end and transmit information indicating the user's exercise end to the electronic device. For example, the first processor 320 may detect the user's exercise end if the exercise data is not collected even after the first certain time for detecting the user's rest action has elapsed while collecting the exercise data.

According to an embodiment, if the first processor 320 receives the exercise routine from the electronic device (e.g., the electronic device 401 of FIG. 2), the first processor 320 may display the exercise routine through the first display 160.

According to an embodiment, if the execution of the exercise routine selected by the user is identified, the first processor 320 may provide guide information through the first display 360 or/and the speaker (not shown) so that the user may exercise according to the exercise routine.

According to an embodiment, the first memory 330 may be implemented to be substantially identical or similar to the memory 130 of FIG. 1.

According to an embodiment, the first display 360 may be implemented in substantially the same or similar manner to the display module 160 of FIG. 1.

In an embodiment, the first display 360 may display an exercise routine received from the electronic device.

According to an embodiment, the first sensor module 376 may be implemented in the same or similar manner to the sensor module 176 of FIG. 1.

According to an embodiment, the first sensor module 376 may include a biometric sensor, a motion sensor, an acceleration sensor, a gyro sensor, an angular velocity sensor, a gyroscope sensor, a heart rate sensor, a temperature/humidity sensor, a barometer, and/or a position sensor.

According to an embodiment, the first communication module 390 may be implemented to be substantially identical or similar to the communication module 190 of FIG. 1 and may include a plurality of communication circuits using different communication technologies.

According to an embodiment, the first communication module 390 may include at least one of a wireless LAN module (not illustrated) and a short-range communication module (not illustrated), and may include an ultra-wide band (UWB) communication module, a Wi-Fi communication module, an NFC communication module, a Bluetooth legacy communication module, and/or a BLE communication module as the short-range communication module (not illustrated).

FIG. 4 is a block diagram 400 illustrating an electronic device according to an embodiment.

Referring to FIG. 4, according to an embodiment, an electronic device 401 (e.g., the electronic device 401 of FIG. 2) may include a second processor 420, second memory 430, a second display 460, a second sensor module 476, and a second communication module 490.

At least some of the components of the electronic device 401 illustrated in FIG. 4 may be identical or similar to the components of the electronic device 101 of FIG. 1, and redundant descriptions will be omitted below.

According to an embodiment, the first processor 420 may be implemented to be substantially identical or similar to the processor 120 of FIG. 2.

According to an embodiment, if the second processor 420 receives exercise data corresponding to the plurality of exercise actions performed by the user wearing the wearable electronic device from the wearable electronic device (e.g., the wearable electronic device 301 of FIG. 2) communicatively connected through the second sensor module 476, the second processor 420 may generate an exercise routine of the user based on the exercise data.

According to an embodiment, if the second processor 420 receives information indicating the user's exercise start from the wearable electronic device, it may wait to receive exercise data.

According to an embodiment, if the second processor 420 receives information indicating the user's exercise end from the wearable electronic device while receiving exercise data from the wearable electronic device, the user's exercise routine may be generated based on exercise data received from the start of the exercise to the end of the exercise.

According to an embodiment, the second processor 420 may detect an exercise type, an exercise time for each exercise type, and a number of times of exercise for each exercise type performed by the user wearing the wearable electronic device based on the exercise data, and generate the user's exercise routine including the detected exercise type, exercise time, and a number of times of exercise number.

According to an embodiment, the second processor 420 may detect a periodic repetitive exercise pattern based on the exercise data, perform grouping based on the periodic repetitive exercise pattern, and classify the type of exercise per grouping using training data.

According to an embodiment, the second processor 420 may pre-process the exercise data, detect a periodical repetitive exercise pattern generation period, and detect a feature using a prediction discrimination analysis technique such as a principal component analysis (PCA) or a liner discriminant analysis (LDA) in the periodical repetitive exercise pattern generation period. If the repetitive exercise pattern section is not detected for more than a threshold time, the second processor 420 may perform clustering on the repetitive exercise patterns using unsupervised learning (e.g., K-means, a Gaussian mixture model (GMM), a hierarchical agglomerative clustering (HAC), or DBSCAN). The second processor 420 may measure similarity by calculating the coefficient of Pearson correlation, Euclidean distance, or cosine similarity, and perform grouping for each cluster having a high similarity. The second processor 420 may classify the type of exercise per grouping using a machine learning-based classifier learning model. The second processor 420 may detect the exercise time and the number of times of exercise, which are detailed information for each of the classified exercise groups.

According to an embodiment, the second processor 420 may detect the rest time and the number of times of rest when the user has rested between the plurality of exercise actions performed by the user based on the rest data received from the wearable electronic device, and may generate the exercise routine by including the detected rest time and number of times of rest in the exercise routine.

According to an embodiment, the second processor 420 may transmit the generated exercise routine to the wearable electronic device so that the generated exercise routine may be provided from the wearable electronic device.

According to an embodiment, the second processor 420 may recommend a change of at least one of the type of exercise, exercise time, number of times of exercise, rest time, or number of times of rest included in the exercise routine according to the user's body information.

According to an embodiment, while visually displaying the type of exercise, exercise time, number of times of exercise, rest time, or number of times of rest included in the exercise routine on the second display 460, the second processor 420 may recommend a change of at least one of the type of exercise, exercise time, number of times of exercise, rest time, or number of times of rest included in the exercise routine according to the user's body information.

For example, if the user's heart rate is higher than a threshold in a first period when first exercise is performed 15 times for one minute and 15 seconds per set when three sets of first exercise (e.g., strength exercise A) among a plurality of exercises are performed, the second processor 420 may recommend to meet the exercise goal that the user may perform with the exercise routine by increasing the number of times of exercise from three sets which are the number of times of exercise in the first period to four sets and decreasing the exercise time to 50 seconds per set and increasing the rest time between the sets.

For example, if the user's body fat percentage is higher than a threshold or there is a body fat percentage set as a target by the user, and the calorie consumption rate when performing the plurality of exercises included in the exercise routine is not included in the calorie consumption rate for reducing the user's body fat percentage, the second processor 420 may recommend adding aerobic exercise or changing one of the plurality of exercises to aerobic exercise.

According to an embodiment, the second processor 420 may provide an edit function capable of changing, by the user, at least one of the type of exercise, the exercise time, the number of times of exercise, the rest time, or the number of times of rest included in the exercise routine while visually displaying the type of exercise, exercise time, number of times of exercise, rest time, or number of times of rest included in the exercise routine on the second display 460.

According to an embodiment, the second processor 420 may store the generated exercise routine, the exercise routine changed according to the recommendation, or the exercise routine edited by the user in the second memory 430 based on the user's selection.

According to an embodiment, the second processor 420 may identify the exercise routine selected by the user among the plurality of exercise routines stored in the second memory 430 and recommend a change of at least one of the type of exercise, the exercise time, the number of times of exercise, the rest time, or the number of times of rest included in the exercise routine, included in the selected exercise routine according to external environment information and/or the body information (e.g., heart rate, stress, weight, and/or body fat percentage) of the user wearing the wearable electronic device (e.g., the wearable electronic device 301 of FIG. 2) at the time of selecting the exercise routine.

For example, the second processor 420 may recommend a change of at least one of the type of exercise, the exercise time, the number of times of exercise, the rest time, or the number of times of rest included in the exercise routine, included in the selected exercise routine based on, e.g., heart rate, stress, weight, and/or body fat percentage, as the biometric information of the user wearing the wearable electronic device (e.g., the wearable electronic device 301 of FIG. 2).

For example, the second processor 420 may recommend a change of at least one of the type of exercise, the exercise time, the number of times of exercise, the rest time, or the number of times of rest included in the exercise routine, included in the selected exercise routine based on weather information predicted while performing the exercise routine and/or the current weather information, as the external environment information.

For example, the second processor 420 may recommend a change of at least one of the type of exercise, the exercise time, the number of times of exercise, the rest time, or the number of times of rest included in the exercise routine, included in the selected exercise routine based on exercise information that may be performed in the exercise-related place when the electronic device is located in the exercise-related place (e.g., gym or swimming pool) as the external environment information.

According to an embodiment, if the execution of the exercise routine selected by the user is identified, the second processor 420 may provide guide information through the second display 460 or/and the speaker (not shown) so that the user may exercise according to the exercise routine.

According to an embodiment, the first memory 430 may be implemented to be substantially identical or similar to the memory 130 of FIG. 2.

According to an embodiment, the second memory 430 may store at least one exercise routine.

According to an embodiment, the first display 460 may be implemented in substantially the same or similar manner to the display module 160 of FIG. 2.

According to an embodiment, the second display 460 may display the exercise routine generated based on the exercise data received from the wearable electronic device (e.g., the wearable electronic device 301 of FIG. 2).

According to an embodiment, the second display 460 may display the exercise routine generated based on the exercise data received from the wearable electronic device (e.g., the wearable electronic device 301 of FIG. 2).

According to an embodiment, while displaying the type of exercise, exercise time, number of times of exercise, rest time, or number of times of rest included in the exercise routine, the second display 460 may display a recommendation routine in which at least one of the type of exercise, exercise time, number of times of exercise, rest time, or number of times of rest included in the exercise routine is changed according to the user's body information.

According to an embodiment, the second display 460 may display an edit function capable of changing, by the user, at least one of the type of exercise, the exercise time, the number of times of exercise, the rest time, or the number of times of rest included in the exercise routine while displaying the type of exercise, exercise time, number of times of exercise, rest time, or number of times of rest included in the exercise routine.

According to an embodiment, the first sensor module 476 may be implemented in the same or similar manner to the sensor module 176 of FIG. 2.

According to an embodiment, the first communication module 490 may be implemented to be substantially identical or similar to the communication module 190 of FIG. 2 and may include a plurality of communication circuits using different communication technologies.

According to an embodiment, the second communication module 490 may include at least one of a wireless LAN module (not illustrated) and a short-range communication module (not illustrated), and may include an ultra-wide band (UWB) communication module, a Wi-Fi communication module, an NFC communication module, a Bluetooth legacy communication module, and/or a BLE communication module as the short-range communication module (not illustrated).

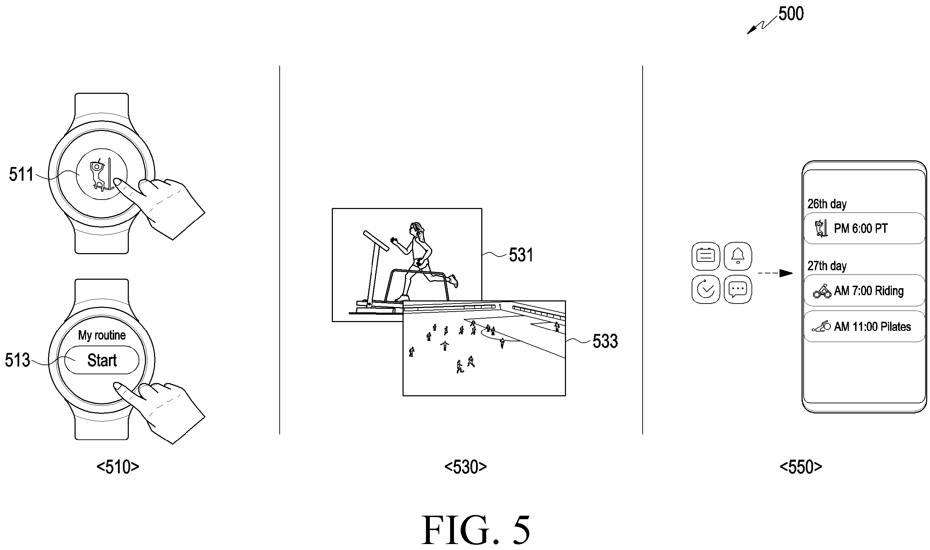

FIG. 5 is a view 500 illustrating an operation of detecting exercise start on a wearable electronic device according to an embodiment.

Referring to FIG. 5, as shown in the screen <510>, the wearable electronic device (e.g., the wearable electronic device 301 of FIG. 2 and/or the wearable electronic device 301) may detect the user's exercise start if receiving a selection of an exercise icon 511 indicating the type of exercise among the plurality of exercise types provided from the wearable electronic device or receiving the user's selection on the start icon 513 indicating a request for generating an exercise routine.

As shown in the screen <530>, the wearable electronic device (e.g., the wearable electronic device 301 of FIG. 2 and/or the wearable electronic device 301 of FIG. 3) may be located in a gym 513 or swimming pool 533 as the exercise-related place and, as shown in the screen <550>, the wearable electronic device (e.g., the wearable electronic device 301 of FIG. 2 and/or the wearable electronic device 301 of FIG. 3) may detect the exercise schedule on the calendar application, notification application, and/or message application stored in the wearable electronic device or the electronic device (e.g., the electronic device (e.g., the electronic device of FIG. 2 and/or the electronic device 401 of FIG. 4)) communicatively connected with the wearable electronic device, store the detected exercise schedule, and detect the start time of the exercise schedule as the user's exercise start time.

The wearable electronic device (e.g., the wearable electronic device 301 of FIG. 2 and/or the wearable electronic device 301 of FIG. 3) may detect the exercise schedule on the calendar application, notification application, and/or message application stored in the wearable electronic device or the electronic device, store the detected exercise schedule and, if exercise data is collected as the exercise action of the user wearing the wearable electronic device when the current time is the start time of the exercise schedule, detect the user's exercise start.

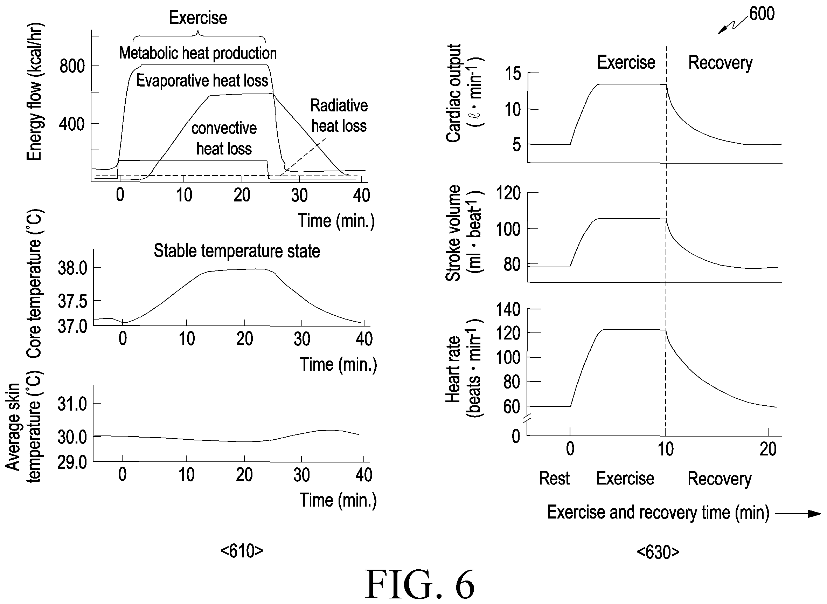

FIG. 6 is a view 600 illustrating an exercise action and a rest action on a wearable electronic device according to an embodiment.

The screen <610> shows graphs of energy flow, core temperature, and average skin temperature while the user wearing the wearable electronic device (e.g., the wearable electronic device 301 of FIG. 2 and/or the wearable electronic device 301 of FIG. 3) does an exercise.

The screen <630> shows graphs of the cardiacoutput, stroke volume, and heart rate when the user wearing the wearable electronic device (e.g., the wearable electronic device 301 of FIG. 2 and/or the wearable electronic device 301 of FIG. 3) does an exercise or recovery.

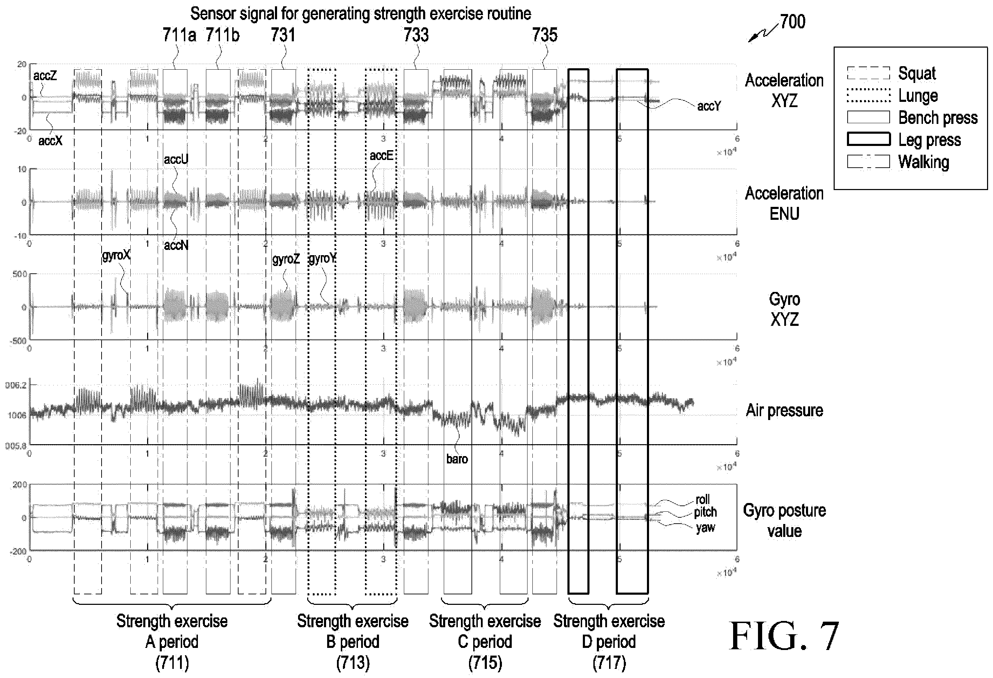

FIG. 7 is a view 700 illustrating an operation of classifying an exercise type based on exercise data on an electronic device according to an embodiment.

Referring to FIG. 7, the electronic device (e.g., the electronic device 401 of FIG. 2 and/or the electronic device 401 of FIG. 4) may detect a periodic repetitive exercise pattern generation section based on the exercise data received from the communicatively connected wearable electronic device (e.g., the wearable electronic device 301 of FIG. 2 and/or the wearable electronic device 301 of FIG. 3), detect a feature using the prediction discrimination analysis technique in the periodic repetitive exercise pattern generation section, perform clustering on the repetitive exercise patterns using unsupervised learning, and perform grouping for each cluster with a high similarity, like "strength exercise A period 711", "strength exercise B period 713", "strength exercise C period 715", and "strength exercise D period 717".

The electronic device may classify the exercise types as a result of classifying per grouping using a machine learning-based classifier learning model, such as "squat period" which is vertical exercise as "strength exercise A period 711", "lunge period" which is horizontal exercise as "strength exercise B period 713", "bench press period" which is vertical exercise as "strength exercise C period 715", and "leg press period" which has little wrist movement as strength exercise D period 717".

In the case of the "squat period", the electronic device may additionally detect the posture information about the wearable electronic device received from the wearable electronic device, as being a representative vertical exercise in which the display of the wearable electronic device (e.g., a watch) worn on the user's wrist faces up, and classify the exercise type.

In the case of the "bench press period", the electronic device may additionally detect the posture information about the wearable electronic device received from the wearable electronic device, as being a representative vertical exercise in which the crown of the wearable electronic device (e.g., a watch) worn on the user's wrist faces up, and classify the exercise type.

The electronic device may detect rest periods 731, 733, and 735 between the exercises based on the rest data received from the wearable electronic device.

When detecting the rest periods 711a and 711b as unspecified and/or irregular times in the "strength exercise A period 711", the electronic device may delete the rest periods 711a and 711b detected as the unspecified and/or irregular periods.

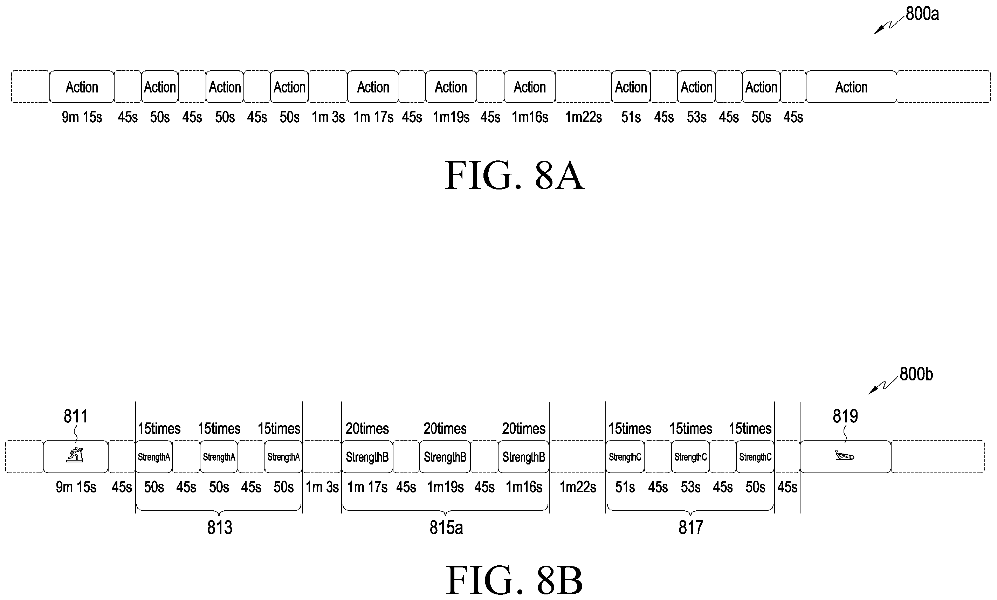

FIGS. 8A to 8C are views 800a to 800c illustrating an operation of generating an exercise routine and recommending an exercise routine based on user's body information on an electronic device according to an embodiment.

As shown in FIG. 8A, the electronic device (e.g., the electronic device 401 of FIG. 2 and/or the electronic device 401 of FIG. 4) may record exercise data and rest data received from a communicatively connected wearable electronic device (e.g., the electronic device 301 of FIG. 2 and/or the wearable electronic device 301 of FIG. 3),

As shown in FIG. 8B, the electronic device (e.g., the electronic device 401 of FIG. 2 and/or the electronic device 401 of FIG. 4) may generate an exercise routine classified into a running period 811, a strength A period 813, a strength B period 815a, a strength C period 817, and a rowing period 819 for each type of exercise based on the exercise data recorded in FIG. 8A, and visually provide the generated exercise routine through the display (e.g., the second display 460 of FIG. 4).

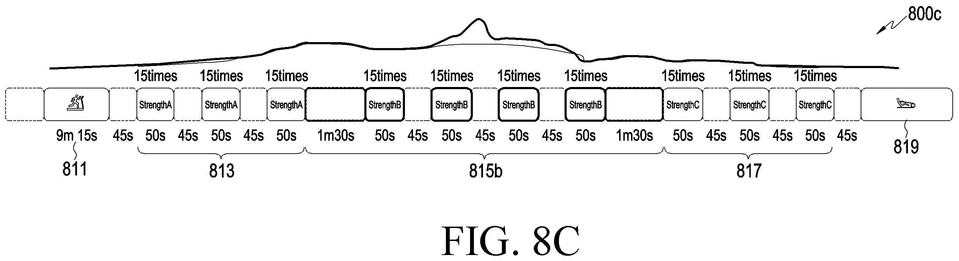

As shown in FIG. 8C, the electronic device (e.g., the electronic device 401 of FIG. 2 and/or the electronic device 401 of FIG. 4) may recommend to meet the exercise goal that the user may perform with the exercise routine by increasing the number of times of exercise from three sets which is the number of times of exercise in the strength B period to four sets and decreasing the exercise time to 50 seconds per set ad increasing the rest time after the start and end of the strength B period when the user's heart rate in the strength B period 815 is higher than a threshold.

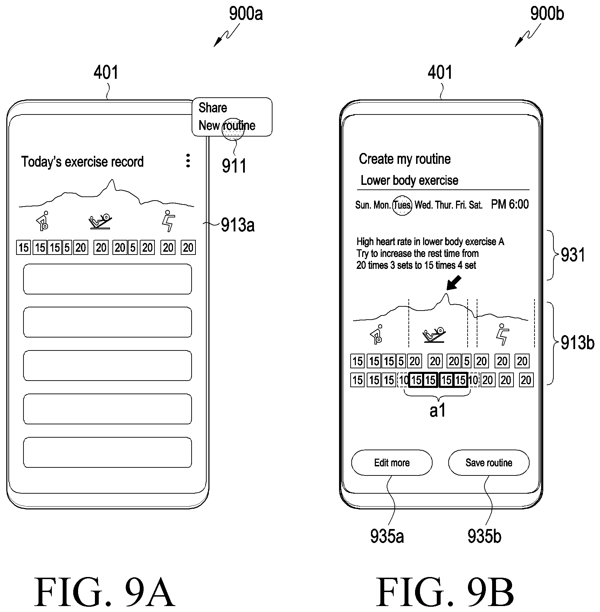

FIGS. 9A to 9D are views 900a to 900D illustrating an operation of editing an exercise routine in an electronic device according to an embodiment.

As shown in FIG. 9A, the electronic device 401 (e.g., the electronic device 401 of FIG. 2 and/or the electronic device 401 of FIG. 4) may provide an exercise routine 913a generated based on exercise data and rest data received from a communicatively connected wearable electronic device (e.g., the electronic device 301 of FIG. 2 and/or the wearable electronic device 301 of FIG. 3). If the selection of "New routine" 911 for recommendation editing and user editing is identified while displaying the exercise routine 913a, it may switch to the mode for recommendation editing for the exercise routine as shown in FIG. 9B.

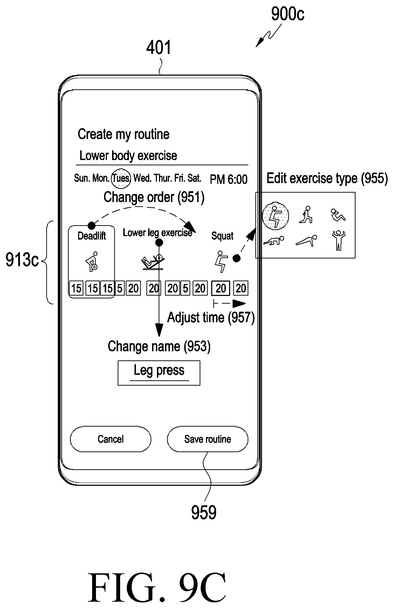

As shown in FIG. 9B, the electronic device 401 (e.g., the electronic device 401 of FIG. 2 and/or the electronic device 401 of FIG. 4) may display an exercise routine 913b in which the lower body exercise A period is changed along with a phrase 931 recommending a change of the exercise time and number of times as the heart rate is increased by more than a threshold during the lower body exercise A period while displaying the exercise routine 913a on the display of the electronic device (e.g., the electronic device 401 of FIG. 4). Upon identifying the selection of "Save routine" 935a, the electronic device 401 may additionally store the exercise routine 913b changed by the recommendation of the electronic device 401 with the name (e.g., lower body exercise) of the exercise routine input by the user. Upon identifying the selection of "Edit more" 935b, the electronic device 401 may switch to a mode for user editing for the exercise routine as shown in FIG. 9C.

As shown in FIG. 9C, the electronic device 401 (e.g., the electronic device 401 of FIG. 2 and/or the electronic device 401 of FIG. 4) may provide a function 951 for changing the order of exercise by the user, a function 953 for changing the name of the exercise, a function 955 for changing the type of exercise, and a function 957 for changing the exercise time while displaying the exercise routine 913a on the display (e.g., the second display 460 of FIG. 4) of the electronic device. Upon identifying the selection of "Save routine" 959, the electronic device 401 may additionally store the exercise routine 913c changed by the user of the electronic device 401 with the name (e.g., lower body exercise) of the exercise routine input by the user.

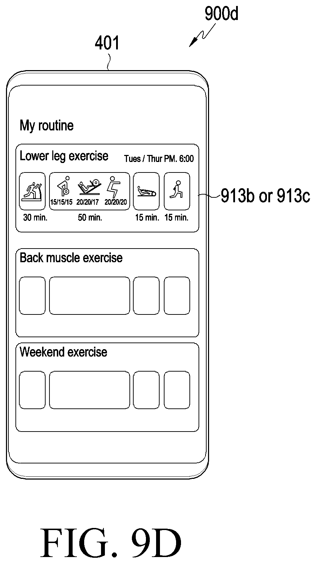

As shown in FIG. 9D, when additionally storing the exercise routine 913b changed by the recommendation of the electronic device 401 as shown in FIG. 9B with the name (e.g., lower body exercise) of the exercise routine input by the user or additionally storing the exercise routine 913c changed by the user as shown in FIG. 9C with the name (e.g., lower body exercise) of the exercise routine input by the user, the electronic device 401 (e.g., the electronic device 401 of FIG. 2 and/or the electronic device 401 of FIG. 4) may store the additionally stored exercise routine 913b or 913c as the user's customized exercise routine.

The electronic device 401 may set and store the name of the exercise routine, the date (e.g., the day of the week) when the exercise routine is performed, and a repetition period of the exercise routine according to the user's input.

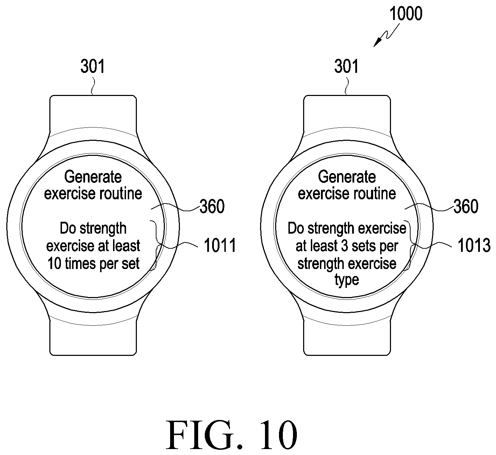

FIG. 10 is a view 1000 illustrating an operation of providing a guide while executing an exercise routine on a wearable electronic device according to an embodiment.

Referring to FIG. 10, the wearable electronic device 301 (e.g., the wearable electronic device 301 of FIG. 2 and the wearable electronic device 301 of FIG. 3) may provide guide information 1011 or 1013 through a display (e.g., the first display 360 of FIG. 3) or/and a speaker (not shown) so that the user may exercise according to the exercise routine if execution of the exercise routine selected by the user is identified.

The system for generating an exercise routine according to an embodiment may include the wearable electronic device (301 in FIG. 2) configured to collect exercise data corresponding to a plurality of exercise actions performed by the user based on the user's biometric information obtained from the sensor module (376 in FIG. 3) of the wearable electronic device and transmit the collected exercise data to the electronic device.

The system according to an embodiment may include an electronic device (401 of FIG. 2; 401 of FIG. 4) configured to generate the user's exercise routine based on the exercise data received from the wearable electronic device and recommend a change of at least one of a type of exercise, an exercise time, a number of times of exercise, a rest time, or a number of times of rest included in the exercise routine according to the user's body information.

In the system according to an embodiment, the wearable electronic device (301 of FIG. 2; 301 of FIG. 3) configured to detect the user's exercise start based on at least one of reception of the user's selection indicating an exercise start, identification of an exercise schedule stored in the wearable electronic device, detection of the wearable electronic device being positioned in an exercise-related place, or detection of the user repeatedly performing the same action.

In the system according to an embodiment, the wearable electronic device (301 of FIG. 2; 301 of FIG. 3) configured to collect rest data corresponding to a rest action performed by the user based on the biometric information while collecting the exercise data and transmit the rest data to the electronic device.

In the system according to an embodiment, the electronic device (401 of FIG. 2; 401 of FIG. 4) configured to detect the type of exercise, the exercise time, and the number of times of exercise based on the exercise data, detect the rest time and the number of times of rest based on the rest data, and generate the user's exercise routine based on the detected type of exercise, exercise time, number of times of exercise, rest time, and number of times of rest.

In the system according to an embodiment, the electronic device (401 of FIG. 2; 401 of FIG. 4) configured to detect a periodic repeated exercise pattern based on the exercise data, perform grouping based on the periodic repeated exercise pattern, and classify the type of exercise per grouping using training data.

In the system according to an embodiment, the electronic device (401 of FIG. 2; 401 of FIG. 4) configured to recommend the change of at least one of the type of exercise, the exercise time, the number of times of exercise, the rest time, or the number of times of rest included in the exercise routine based on the user's body information while visually displaying the exercise routine on a display of the electronic device.

In the system according to an embodiment, the electronic device (401 of FIG. 2; 401 of FIG. 4) configured to transmit the exercise routine to the wearable electronic device to display the exercise routine on a display of the wearable electronic device.

In the system according to an embodiment, the electronic device (401 of FIG. 2; 401 of FIG. 4) configured to provide an edit function in which at least one of the type of exercise, the exercise time, the number of times of exercise, the rest time, or the number of times of rest included in the exercise routine is changeable by the user while visually displaying the exercise routine on the display of the electronic device.

In the system according to an embodiment, the electronic device (401 of FIG. 2; 401 of FIG. 4) configured to recommend the change of at least one of the type of exercise, the exercise time, the number of times of exercise, the rest time, or the number of times of rest included in the exercise routine according to the user's body information at a time of selecting execution of the exercise routine when identifying selection for the execution of the exercise routine.

In the system according to an embodiment, the electronic device (401 of FIG. 2; 401 of FIG. 4) configured to recommend the change of at least one of the type of exercise, the exercise time, the number of times of exercise, the rest time, or the number of times of rest included in the exercise routine according to external environment information at a time of selecting execution of the exercise routine when identifying selection for the execution of the exercise routine.

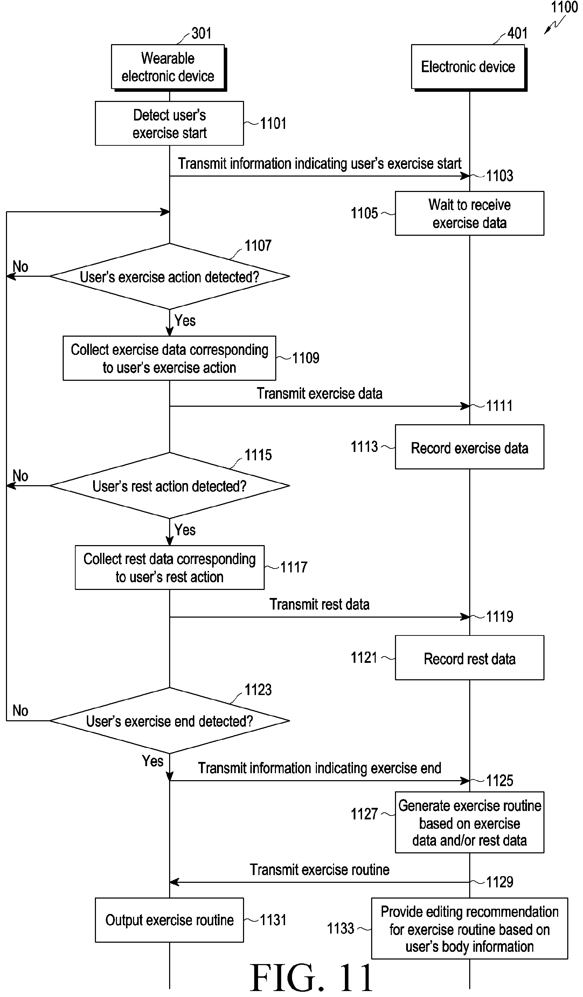

FIG. 11 is a flowchart 1100 illustrating an operation for generating an exercise routine according to an embodiment. The operations for generating an exercise routine may include operations 1101 to 1133. **In** the following embodiments, each operation may be performed sequentially, but is not necessarily performed sequentially. For example, the order of the operations may be changed, and at least two operations may be performed in parallel or other operations may be added.

**In** operation 1101, the wearable electronic device 301 (e.g., the wearable electronic device 301 of FIG. 2 and/or the wearable electronic device 301 of FIG. 3) may detect the user's exercise start.

According to an embodiment, if receiving the user's selection indicating the start of the exercise, the wearable electronic device 301 may detect the user's exercise start.

According to an embodiment, if identifying an exercise schedule stored in the wearable electronic device or the electronic device, the wearable electronic device 301 may detect the user's exercise start.

According to an embodiment, if the wearable electronic device 301 is located in an exercise-related place, the wearable electronic device 301 may detect the user's exercise start.

According to an embodiment, the wearable electronic device 301 may detect the user's exercise start if detecting that the user wearing the wearable electronic device 301 repeatedly performs the same action.

In operation 1103, the wearable electronic device 301 (e.g., the wearable electronic device 301 of FIG. 2 and/or the wearable electronic device 301 of FIG. 3) may transmit information indicating the user's exercise start to the electronic device 401.

According to an embodiment, the wearable electronic device 301 may transmit information indicating the start of the exercise to the electronic device 401 through the communication module (e.g., the first communication module 390 of FIG. 3) of the wearable electronic device.

In operation 1105, the electronic device 401 (e.g., the electronic device 401 of FIG. 2 and/or the electronic device of FIG. 4) may wait to receive exercise data.

According to an embodiment, if the electronic device 401 receives information indicating the start of exercise from the wearable electronic device 301, it may wait to receive the exercise data.

In operation 1107, the wearable electronic device 301 (e.g., the wearable electronic device 301 of FIG. 2 and/or the wearable 301 of FIG. 3) may collect exercise data corresponding to the user's exercise action in operation 1109 if detecting the user's exercise action.

According to an embodiment, the wearable electronic device 301 may detect the user's exercise action based on biometric information obtained through the sensor module (e.g., the first sensor module 376 of FIG. 3) of the wearable electronic device, and collect biometric information obtained through the sensor module during the exercise action as exercise data.

In operation 1111, the wearable electronic device 301 (e.g., the wearable electronic device 301 of FIG. 2 and/or the wearable 301 of FIG. 3) may transmit exercise data to the electronic device 401.

According to an embodiment, the wearable electronic device 301 may transmit the exercise data to the electronic device 401 through the communication module (e.g., the first communication module 390 of FIG. 3) of the wearable electronic device.

In operation 1113, the electronic device 401 (e.g., the electronic device 401 of FIG. 2 and/or the electronic device 401 of FIG. 4) may record exercise data.

According to an embodiment, the electronic device 401 may record exercise data received from the wearable electronic device.

In operation 1115, if detecting the user's rest action, the wearable electronic device 301 (e.g., the wearable electronic device 301 of FIG. 2 and/or the wearable 301 of FIG. 3) may collect rest data corresponding to the user's rest action in operation 1117.

According to an embodiment, when the same exercise data corresponding to the exercise action is not collected within a first predetermined time (e.g., 30 minutes) while collecting exercise data corresponding to the user's exercise action, and if the user's heart rate detected while performing the exercise action decreases below a threshold, the wearable electronic device 301 may detect it as the user's rest action and collect rest data including the rest time and/or the number of times of rest performed.

In operation 1119, the wearable electronic device 301 (e.g., the wearable electronic device 301 of FIG. 2 and/or the wearable 301 of FIG. 3) may transmit rest data to the electronic device 401.

According to an embodiment, the wearable electronic device 301 may transmit the rest data to the electronic device 401 through the communication module (e.g., the first communication module 390 of FIG. 3) of the wearable electronic device.

In operation 1121, the electronic device 401 (e.g., the electronic device 401 of FIG. 2 and/or the electronic device of FIG. 4) may record rest data.

According to an embodiment, the electronic device 401 may record rest data received from the wearable electronic device.

If detecting the user's exercise end in operation 1123, the wearable electronic device 301 (e.g., the wearable electronic device 301 of FIG. 2 and/or the wearable 301 of FIG. 3) may transmit information indicating the end of the exercise to the electronic device 401 in operation 1125.

According to an embodiment, if the exercise data is not collected even after the first predetermined time for detecting the user's rest action elapses while collecting the exercise data and transmitting the collected exercise data to the electronic device 401, the wearable electronic device 301 may detect the user's exercise end.

According to an embodiment, the wearable electronic device 301 may transmit information indicating the end of the exercise to the electronic device 401 through the communication module (e.g., the first communication module 390 of FIG. 3) of the wearable electronic device.

In operation 1127, the electronic device 401 (e.g., the electronic device 401 of FIG. 2 and/or the electronic device 401 of FIG. 4) may generate an exercise routine based on exercise data and/or rest data.

According to an embodiment, if the electronic device 401 receives information indicating the end of the exercise from the wearable electronic device, the electronic device 401 may generate an exercise routine based on exercise data recorded from the time when the information indicating the start of the exercise is received to the time of indicating the end of the exercise.

According to an embodiment, if the electronic device 401 receives information indicating the user's exercise end from the wearable electronic device while receiving exercise data from the wearable electronic device, it may generate the user's exercise routine based on the exercise data received from the exercise start to the exercise end.

According to an embodiment, the electronic device 401 may pre-process the exercise data, detect a periodical repetitive exercise pattern generation section, and detect a feature using a prediction discrimination analysis technique such as a principal component analysis (PCA) or a liner discriminant analysis (LDA) in the periodical repetitive exercise pattern generation section. If the repetitive exercise pattern section is not detected for more than a threshold time, the electronic device 401 may perform clustering on the repetitive exercise patterns using unsupervised learning (e.g., K-means, a Gaussian mixture model (GMM), a hierarchical agglomerative clustering (HAC), or DBSCAN). The electronic device 401 may measure similarity by calculating the coefficient of Pearson correlation, Euclidean distance, or cosine similarity, and perform grouping for each cluster having a high similarity. The electronic device 401 may classify the type of exercise per grouping using a machine learning-based classifier learning model. The electronic device 401 may detect the exercise time and the number of times of exercise, which are detailed information for each of the classified exercise groups.

According to an embodiment, the electronic device 401 may detect the rest time and the number of times of rest by the user between the plurality of exercise actions performed by the user based on rest data recorded from the time when information indicating the exercise start is received to the time of indicating the exercise end, and may generate the exercise routine by including the detected rest time and number of times of rest in the exercise routine.

In operation 1129, the electronic device 401 (e.g., the electronic device 401 of FIG. 2 and/or the electronic device of FIG. 4) may transmit the exercise routine to the wearable electronic device 301.

According to an embodiment, the electronic device 401 may transmit the exercise routine to the wearable electronic device 501 through a communication module (e.g., the second communication module 490 of FIG. 4) of the electronic device.

In operation 1131, the wearable electronic device 301 (e.g., the wearable electronic device 301 of FIG. 2 and/or the wearable 301 of FIG. 3) may output the exercise routine

According to an embodiment, if the wearable electronic device 301 receives the exercise routine from the electronic device 401, it may display the exercise routine through the display (e.g., the first display 360 of FIG. 3) of the wearable electronic device.

**In** operation 1133, the electronic device 401 (e.g., the electronic device 401 of FIG. 2 and/or the electronic device of FIG. 4) may provide an edit recommendation for the exercise routine based on the user's body information.

According to an embodiment, while visually displaying the type of exercise, exercise time, number of times of exercise, rest time, or number of times of rest included in the exercise routine on the display (e.g., the second display 460 of FIG. 4) of the electronic device, the electronic device 401 may recommend a change of at least one of the type of exercise, exercise time, number of times of exercise, rest time, or number of times of rest included in the exercise routine according to the user's body information. For example, the user's body information may include the user's heart rate, blood pressure, and stress while performing a plurality of exercise actions, and may include the user's current weight, body fat, and skeletal muscle mass.

According to an embodiment, the electronic device 401 may additionally provide an edit function capable of changing, by the user, at least one of the type of exercise, the exercise time, the number of times of exercise, the rest time, or the number of times of rest included in the exercise routine while visually displaying the type of exercise, exercise time, number of times of exercise, rest time, or number of times of rest included in the exercise routine on the display (e.g., the second display 460 of FIG. 4) of the electronic device.

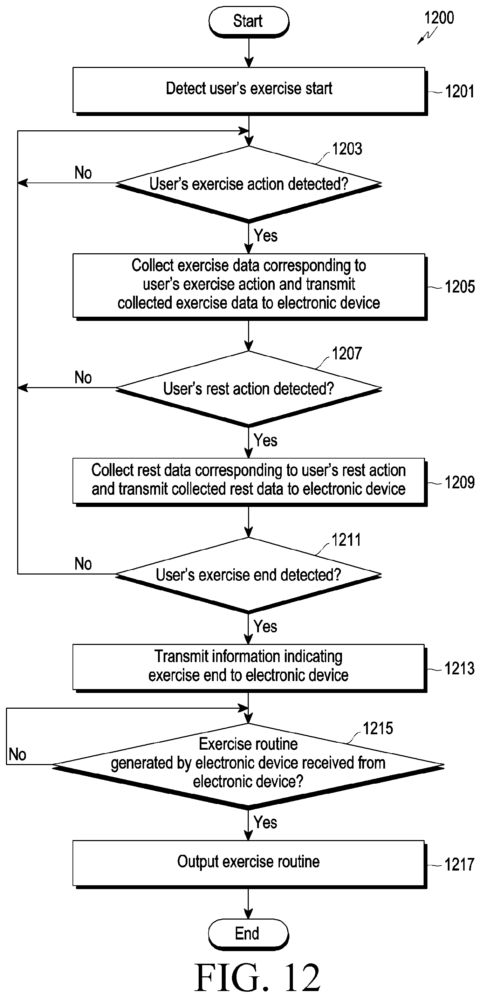

FIG. 12 is a flowchart 1200 illustrating an operation for generating an exercise routine on a wearable electronic device according to an embodiment. The operations for generating an exercise routine may include operations 1201 to 1217. **In** the following embodiments, each operation may be performed sequentially, but is not necessarily performed sequentially. For example, the order of the operations may be changed, and at least two operations may be performed in parallel or other operations may be added.

In operation 1201, the wearable electronic device (e.g., the wearable electronic device 301 of FIG. 2 and/or the wearable electronic device 301 of FIG. 3) may detect the user's exercise start.

According to an embodiment, the wearable electronic device may detect the user's exercise start based on at least one of reception of the user's selection indicating an exercise start, identification of an exercise schedule stored in the wearable electronic device, detection of the wearable electronic device being positioned in an exercise-related place, or detection of the user repeatedly performing the same action.

According to an embodiment, the wearable electronic device may transmit information indicating the user's exercise start to the electronic device through the communication module (e.g., the first communication module 390 of FIG. 3) of the wearable electronic device.

If detecting the user's exercise action in operation 1203, the wearable electronic device (e.g., the wearable electronic device 301 of FIG. 2 and/or the wearable 301 of FIG. 3) may collect exercise data corresponding to the user's exercise action and transmit the collected exercise data to the electronic device (e.g., the electronic device 401 of FIG. 2 and/or the electronic device 401 of FIG. 4) in operation 1205,.

According to an embodiment, the wearable electronic device may detect the user's exercise action based on biometric information obtained through the sensor module (e.g., the first sensor module 376 of FIG. 3) of the wearable electronic device, and collect biometric information obtained through the sensor module during the exercise action as exercise data.

According to an embodiment, the wearable electronic device may transmit the exercise data to the electronic device through the communication module (e.g., the first communication module 390 of FIG. 3) of the wearable electronic device.

If detecting the user's rest action in operation 1207, the wearable electronic device (e.g., the wearable electronic device 301 of FIG. 2 and/or the wearable 301 of FIG. 3) may collect rest data corresponding to the user's rest action and transmit the collected rest data to the electronic device (e.g., the electronic device 401 of FIG. 2 and/or the electronic device 401 of FIG. 4) in operation 1209.

According to an embodiment, when the same exercise data corresponding to the exercise action is not collected within a first predetermined time (e.g., 30 minutes) while collecting exercise data corresponding to the user's exercise action, and if the user's heart rate detected while performing the exercise action decreases below a threshold, the wearable electronic device may detect it as the user's rest action and collect rest data including the rest time and/or the number of times of rest performed.

According to an embodiment, the wearable electronic device may transmit the rest data to the electronic device through the communication module (e.g., the first communication module 390 of FIG. 3) of the wearable electronic device.

When detecting the user's exercise end in operation 1211, the wearable electronic device 301 (e.g., the wearable electronic device 301 of FIG. 2 and/or the wearable 301 of FIG. 3) may transmit information indicating the end of the exercise to the electronic device (e.g., the electronic device 401 of FIG. 2 and/or the electronic device 401 of FIG. 4) in operation 1213.

According to an embodiment, the wearable electronic device may detect the user's exercise end if the exercise data is not collected even after a first predetermined time has elapsed for detecting the user's rest action while collecting the exercise data.

According to an embodiment, the wearable electronic device may transmit information indicating the end of the exercise to the electronic device through a communication module (e.g., the first communication module 390 of FIG. 3) of the wearable electronic device.

In operation 1215, the wearable electronic device 301 (e.g., the wearable electronic device 301 of FIG. 2 and/or the wearable 301 of FIG. 3) may output the exercise routine in operation 1217, if receiving the exercise routine generated by the electronic device from the electronic device (e.g., the electronic device 401 of FIG. 2 and/or the electronic device 401 of FIG. 4).

According to an embodiment, the wearable electronic device may output the exercise routine received from the electronic device through the display (e.g., the first display 360 of FIG. 3) of the wearable electronic device.

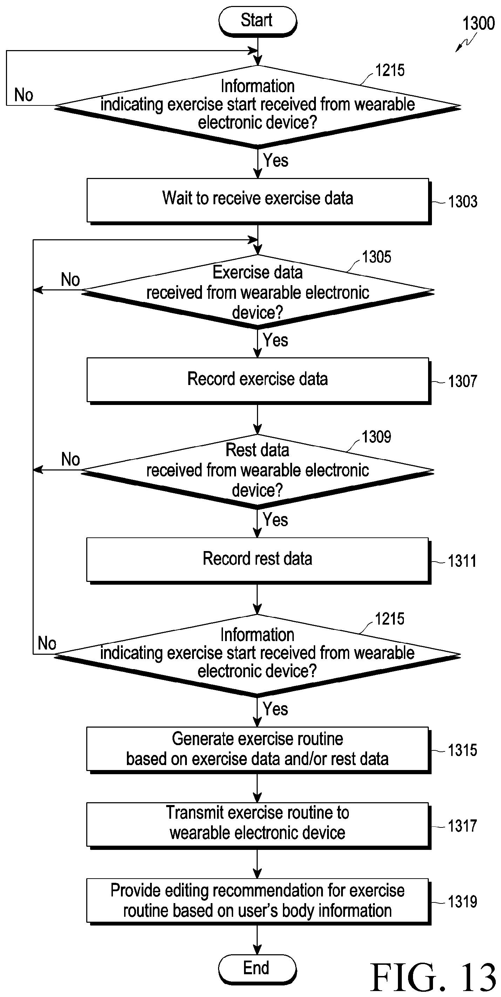

FIG. 13 is a flowchart 1300 illustrating an operation for generating an exercise routine on an electronic device according to an embodiment. The operations for generating an exercise routine may include operations 1301 to 1319. In the following embodiments, each operation may be performed sequentially, but is not necessarily performed sequentially. For example, the order of the operations may be changed, and at least two operations may be performed in parallel or other operations may be added.

If the electronic device (e.g., the electronic device 401 of FIG. 2 and/or the electronic device of FIG. 4) receives information indicating the start of exercise from the wearable electronic device (e.g., the wearable electronic device 301 of FIG. 2 and/or the wearable electronic device 301 of FIG. 3) in operation 1301, it may wait to receive exercise data in operation 1303.

According to an embodiment, if the electronic device receives information indicating an exercise start from the communicatively connected wearable electronic device, the electronic device may wait to receive the exercise data.

In operation 1305, if the electronic device (e.g., the electronic device 401 of FIG. 2 and/or the electronic device of FIG. 4) receives exercise data from the wearable electronic device (e.g., the wearable electronic device 301 of FIG. 2 and/or the wearable electronic device 301 of FIG. 3), it may record the received exercise data in operation 1307.

According to an embodiment, the electronic device may record exercise data received from the wearable electronic device.

In operation 1309, if the electronic device (e.g., the electronic device 401 of FIG. 2 and/or the electronic device of FIG. 4) receives rest data from the wearable electronic device (e.g., the wearable electronic device 301 of FIG. 2 and/or the wearable electronic device 301 of FIG. 3), it may record the received rest data in operation 1311.

If the electronic device 401 (e.g., the electronic device 401 of FIG. 2 and/or the electronic device 401 of FIG. 4) receives information indicating the end of the exercise from the wearable electronic device (e.g., the wearable electronic device 301 of FIG. 2 and/or the wearable electronic device 301 of FIG. 3) in operation 1313, it may generate an exercise routine based on exercise data and/or rest data in operation 1315.

According to an embodiment, if the electronic device receives information indicating the end of the exercise from the wearable electronic device, the electronic device 401 may generate an exercise routine based on exercise data recorded from the time when the information indicating the start of the exercise is received to the time of indicating the end of the exercise.

According to an embodiment, the electronic device may detect an exercise type, an exercise time for each exercise type, and a number of times of exercise for each exercise type performed by the user wearing the wearable electronic device based on the exercise data, and generate the user's exercise routine including the detected exercise type, exercise time, and a number of times of exercise number.

According to an embodiment, the electronic device may detect a periodic repetitive exercise pattern based on the exercise data, perform grouping based on the periodic repetitive exercise pattern, and classify the type of exercise per grouping using training data. The electronic device may detect the exercise time and the number of times of exercise, which are detailed information for each of the classified exercise groups.

According to an embodiment, the electronic device may detect the rest time and the number of times of rest by the user between the plurality of exercise actions performed by the user based on rest data recorded from the time when information indicating the exercise start is received to the time of indicating the exercise end, and may generate the exercise routine by including the detected rest time and number of times of rest in the exercise routine.

In operation 1317, the electronic device (e.g., the electronic device 401 of FIG. 2 and/or the electronic device 401 of FIG. 4) may transmit the exercise routine to the wearable electronic device (e.g., the wearable electronic device 301 of FIG. 2 and/or the wearable electronic device 301 of FIG. 3).

According to an embodiment, the electronic device may transmit the exercise routine to the wearable electronic device 501 through a communication module (e.g., the second communication module 490 of FIG. 4) of the electronic device.

In operation 1319, the electronic device (e.g., the electronic device 401 of FIG. 2 and/or the electronic device of FIG. 4) may provide an edit recommendation for the exercise routine based on the user's body information.

According to an embodiment, while visually displaying the type of exercise, exercise time, number of times of exercise, rest time, or number of times of rest included in the exercise routine on the display (e.g., the second display 460 of FIG. 4) of the electronic device, the electronic device may recommend a change of at least one of the type of exercise, exercise time, number of times of exercise, rest time, or number of times of rest included in the exercise routine according to the user's body information.

According to an embodiment, the electronic device may additionally provide an edit function capable of changing, by the user, at least one of the type of exercise, the exercise time, the number of times of exercise, the rest time, or the number of times of rest included in the exercise routine while visually displaying the type of exercise, exercise time, number of times of exercise, rest time, or number of times of rest included in the exercise routine on the display (e.g., the second display 460 of FIG. 4) of the electronic device.

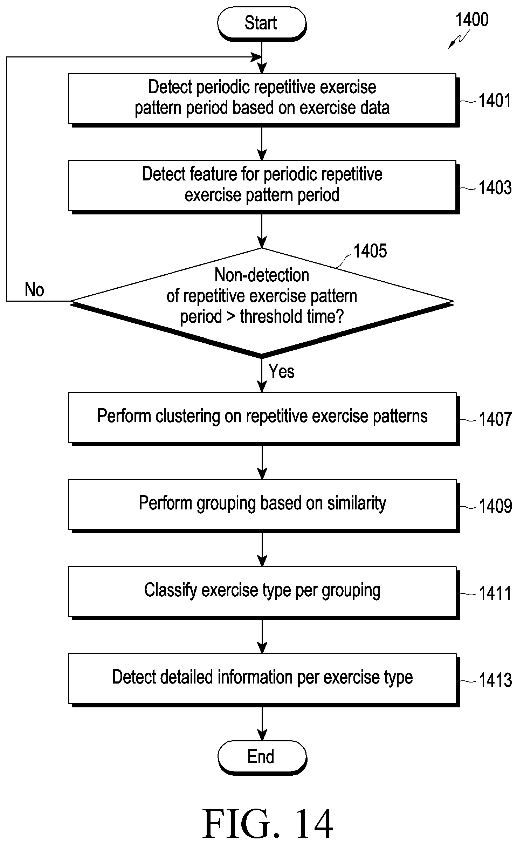

FIG. 14 is a flowchart 1400 illustrating an operation for classifying an exercise type on an electronic device according to an embodiment. The operations for classifying the exercise type may include operations 1401 to 1413. In the following embodiments, each operation may be performed sequentially, but is not necessarily performed sequentially. For example, the order of the operations may be changed, and at least two operations may be performed in parallel or other operations may be added.

In operation 1401, the electronic device (e.g., the electronic device 401 of FIG. 2 and/or the electronic device 401 of FIG. 4) may detect a periodic repetitive exercise pattern period based on the exercise data.

According to an embodiment, the electronic device may detect the periodic repetitive exercise pattern period after pre-processing the exercise data.

In operation 1403, the electronic device (e.g., the electronic device 401 of FIG. 2 and/or the electronic device 401 of FIG. 4) may detect a feature for the periodic repetitive exercise pattern period.

According to an embodiment, the electronic device may detect a feature in the periodic repetitive exercise pattern generation period using a prediction discrimination analysis technique such as a principal component analysis (PCA) or a liner discriminant analysis (LDA).

In operation 1405, the electronic device (e.g., the electronic device 401 of FIG. 2 and/or the electronic device 401 of FIG. 4) may determine whether the non-detection of the repetitive exercise pattern period is larger than or equal to a threshold time.

In operation 1405, if the non-detection of the repetitive exercise pattern period is less than or equal to the threshold time, the electronic device may repeatedly perform operation 1401 to operation 1403.

In operation 1405, if the non-detection of the repetitive exercise pattern period is larger than or equal to the threshold time, the electronic device may perform clustering on repetitive exercise patterns in operation 1407.

According to an embodiment, the electronic device may perform clustering on the repetitive exercise patterns using unsupervised learning (e.g., K-means, a Gaussian mixture model (GMM), a hierarchical agglomerative clustering (HAC), or DBSCAN).

In operation 1409, the electronic device (e.g., the electronic device 401 of FIG. 2 and/or the electronic device 401 of FIG. 4) may perform grouping based on similarity.

According to an embodiment, the electronic device may measure similarity by calculating the coefficient of Pearson correlation, Euclidean distance, or cosine similarity, and perform grouping for each cluster having a high similarity.

In operation 1411, the electronic device (e.g., the electronic device 401 of FIG. 2 and/or the electronic device 401 of FIG. 4) may classify the type of exercise per grouping.

According to an embodiment, the electronic device may select a representative pattern per group and classify the type of exercise per grouping using a machine learning-based classifier learning model.

In operation 1413, the electronic device (e.g., the electronic device 401 of FIG. 2 and/or the electronic device of FIG. 4) may detect detailed information for each type of exercise.

According to an embodiment, the electronic device may detect the exercise time and the number of times of exercise, which are detailed information for each of the classified exercise groups.

A method for generating an exercise routine, according to an embodiment, may comprise collecting, by a wearable electronic device (301 of FIG. 2; 301 of FIG. 3), exercise data corresponding to a plurality of exercise actions performed by a user wearing the wearable electronic device based on biometric information of the user obtained from a sensor module (376 of FIG. 2) of the wearable electronic device and transmitting the collected exercise data to an electronic device.

The method according to an embodiment may comprise generating the user's exercise routine based on the exercise data received from the wearable electronic device, by the electronic device.

The method according to an embodiment may comprise recommending, by the electronic device (401 of FIG. 2; 401 of FIG. 4), the change of at least one of the type of exercise, the exercise time, the number of times of exercise, the rest time, or the number of times of rest included in the exercise routine according to the user's body information.

The method according to an embodiment may further comprise, by the wearable electronic device (301 of FIG. 2; 301 of FIG. 3), detecting the user's exercise start based on at least one of reception of the user's selection indicating an exercise start, identification of an exercise schedule stored in the wearable electronic device, detection of the wearable electronic device being positioned in an exercise-related place, or detection of the user repeatedly performing the same action.

The method according to an embodiment may further comprise, by the wearable electronic device (301 of FIG. 2; 301 of FIG. 3), collecting rest data corresponding to a rest action performed by the user based on the biometric information while collecting the exercise data and transmitting the rest data to the electronic device.

The method according to an embodiment may further comprise, by the electronic device (401 of FIG. 2; 401 of FIG. 4), detecting the type of exercise, the exercise time, and the number of times of exercise based on the exercise data, detecting the rest time and the number of times of rest based on the rest data, and generating the user's exercise routine based on the detected type of exercise, exercise time, number of times of exercise, rest time, and number of times of rest.

The method according to an embodiment may further comprise, by the electronic device (401 of FIG. 2; 401 of FIG. 4), detecting a periodic repetitive exercise pattern based on the exercise data, performing grouping based on the periodic repetitive exercise pattern, and classifying the type of exercise per grouping using training data.

The method according to an embodiment may further comprise recommending, by the electronic device (401 of FIG. 2; 401 of FIG. 4), the change of at least one of the type of exercise, the exercise time, the number of times of exercise, the rest time, or the number of times of rest included in the exercise routine based on the user's body information while visually displaying the exercise routine on a display of the electronic device.

The method according to an embodiment may further comprise, by the electronic device (401 of FIG. 2; 401 of FIG. 4), transmitting the exercise routine to the wearable electronic device to display the exercise routine on a display of the wearable electronic device.

The method according to an embodiment may comprise providing, by the electronic device (401 of FIG. 2; 401 of FIG. 4), an edit function in which at least one of the type of exercise, the exercise time, the number of times of exercise, the rest time, or the number of times of rest included in the exercise routine is changeable by the user while visually displaying the exercise routine on the display of the electronic device.

The method according to an embodiment may comprise recommending, by the electronic device (401 of FIG. 2; 401 of FIG. 4), the change of at least one of the type of exercise, the exercise time, the number of times of exercise, the rest time, or the number of times of rest included in the exercise routine according to the user's body information at a time of selecting execution of the exercise routine when identifying selection for the execution of the exercise routine.

The method according to an embodiment may comprise recommending, by the electronic device (401 of FIG. 2; 401 of FIG. 4), the change of at least one of the type of exercise, the exercise time, the number of times of exercise, the rest time, or the number of times of rest included in the exercise routine according to external environment information at a time of selecting execution of the exercise routine when identifying selection for the execution of the exercise routine.

The electronic device according to an embodiment of the disclosure may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

An embodiment of the disclosure may be implemented as software (e.g., the program 140) including one or more instructions that are stored in a storage medium (e.g., internal memory 136 or external memory 138) that is readable by a machine (e.g., the electronic device 101 or the electronic device 301). For example, a processor (e.g., the processor 520) of the machine (e.g., the electronic device 301) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The storage medium readable by the machine may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to an embodiment of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smartphones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities. Some of the plurality of entities may be separately disposed in different components. According to an embodiment, one or more of the above-described components may be omitted, or one or more other components may be added. Alternatively or additionally, a plurality of components (e.g., modules or programs) may be integrated into a single component. **In** such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

According to an embodiment of the disclosure, in a non-transitional storage medium storing instructions configured to, when executed by an electronic device, enable the electronic device to perform at least one operation, the at least one operation may comprise collecting, by a wearable electronic device, exercise data corresponding to a plurality of exercise actions performed by a user wearing the wearable electronic device based on biometric information of the user obtained from a sensor module of the wearable electronic device and transmitting the collected exercise data to an electronic device and generating the user's exercise routine based on the exercise data received from the wearable electronic device, by the electronic device, and recommending, by the electronic device, the change of at least one of the type of exercise, the exercise time, the number of times of exercise, the rest time, or the number of times of rest included in the exercise routine based on the user's body information while visually displaying the exercise routine on a display of the electronic device.

## Claims

1. A system for generating an exercise routine, comprising:
a wearable electronic device (301 of FIG. 2; 301 of FIG. 3) configured to collect exercise data corresponding to a plurality of exercise actions performed by a user wearing the wearable electronic device based on biometric information of the user obtained from a sensor module (376 of FIG. 3) of the wearable electronic device and transmit the collected exercise data to an electronic device; and
an electronic device (401 of FIG. 2; 401 of FIG. 4) configured to generate the user's exercise routine based on the exercise data received from the wearable electronic device and recommend a change of at least one of a type of exercise, an exercise time, a number of times of exercise, a rest time, or a number of times of rest included in the exercise routine according to the user's body information.

2. The system of claim 1, wherein the wearable electronic device (301 of FIG. 2; 301 of FIG. 3) is configured to detect the user's exercise start based on at least one of reception of the user's selection indicating an exercise start, identification of an exercise schedule stored in the wearable electronic device, detection of the wearable electronic device being positioned in an exercise-related place, or detection of the user repeatedly performing the same action.

3. The system of claim 1 or 2, wherein the wearable electronic device (301 of FIG. 2; 301 of FIG. 3) is configured to collect rest data corresponding to a rest action performed by the user based on the biometric information while collecting the exercise data, and transmit the rest data to the electronic device.

4. The system of any one of claims 1 to 3, wherein the electronic device (401 of FIG. 2; 401 of FIG. 4) is configured to detect the type of exercise, the exercise time, and the number of times of exercise based on the exercise data, detect the rest time and the number of times of rest based on the rest data, and generate the user's exercise routine based on the detected type of exercise, exercise time, number of times of exercise, rest time, and number of times of rest.

5. The system of any one of claims 1 to 4, wherein the electronic device (401 of FIG. 2; 401 of FIG. 4) is configured to detect a periodic repetitive exercise pattern based on the exercise data, perform grouping based on the periodic repetitive exercise pattern, and classify the type of exercise per grouping using training data.

6. The system of any one of claims 1 to 5, wherein the electronic device (401 of FIG. 2; 401 of FIG. 4) is configured to recommend the change of at least one of the type of exercise, the exercise time, the number of times of exercise, the rest time, or the number of times of rest included in the exercise routine based on the user's body information while visually displaying the exercise routine on a display of the electronic device.

7. The system of any one of claims 1 to 6, wherein the electronic device (401 of FIG. 2; 401 of FIG. 4) is configured to transmit the exercise routine to the wearable electronic device to display the exercise routine on a display of the wearable electronic device.

8. The system of any one of claims 1 to 7, wherein the electronic device (401 of FIG. 2; 401 of FIG. 4), while visually displaying the exercise routine on a display of the electronic device, is configured to provide an edit function in which at least one of the type of exercise, the exercise time, the number of times of exercise, the rest time, or the number of times of rest included in the exercise routine is changeable by the user.

9. The system of any one of claims 1 to 8, wherein the electronic device (401 of FIG. 2; 401 of FIG. 4) is configured to recommend the change of at least one of the type of exercise, the exercise time, the number of times of exercise, the rest time, or the number of times of rest included in the exercise routine according to the user's body information at a time of selecting execution of the exercise routine when identifying selection for execution of the exercise routine.

10. The system of any one of claims 1 to 9, wherein the electronic device (401 of FIG. 2; 401 of FIG. 4) is configured to recommend the change of at least one of the type of exercise, the exercise time, the number of times of exercise, the rest time, or the number of times of rest included in the exercise routine according to external environment information at a time of selecting execution of the exercise routine when identifying selection for execution of the exercise routine.

11. A method for generating an exercise routine, the method comprising:
collecting, by a wearable electronic device (301 of FIG. 2; 301 of FIG. 3), exercise data corresponding to a plurality of exercise actions performed by a user wearing the wearable electronic device based on biometric information of the user obtained from a sensor module (376 of FIG. 2) of the wearable electronic device and transmitting the collected exercise data to an electronic device; and
generating the user's exercise routine based on the exercise data received from the wearable electronic device, by the electronic device (401 of FIG. 2; 401 of FIG. 4); and
recommending, by the electronic device (401 of FIG. 2; 401 of FIG. 4), the change of at least one of the type of exercise, the exercise time, the number of times of exercise, the rest time, or the number of times of rest included in the exercise routine based on the user's body information while visually displaying the exercise routine on a display of the electronic device.

12. The method of claim 11, further comprising, by the wearable electronic device (301 of FIG. 2; 301 of FIG. 3), detecting the user's exercise start based on at least one of reception of the user's selection indicating an exercise start, identification of an exercise schedule stored in the wearable electronic device, detection of the wearable electronic device being positioned in an exercise-related place, or detection of the user repeatedly performing the same action.

13. The method of claim 11 or 12, further comprising: by the wearable electronic device (301 of FIG. 2; 301 of FIG. 3), collecting rest data corresponding to a rest action performed by the user based on the biometric information while collecting the exercise data; and
transmitting the rest data to the electronic device.

14. The method of any one of claims 11 to 13, further comprising: by the electronic device (401 of FIG. 2; 401 of FIG. 4),
detecting the type of exercise, the exercise time, and the number of times of exercise based on the exercise data;
detecting the rest time and the number of times of rest based on the rest data; and
generating the user's exercise routine based on the detected type of exercise, exercise time, number of times of exercise, rest time, and number of times of rest.

15. The method of any one of claims 11 to 14, further comprising: by the electronic device (401 of FIG. 2; 401 of FIG. 4),
detecting a periodic repetitive exercise pattern based on the exercise data;
performing grouping based on the periodic repetitive exercise pattern; and
classifying the type of exercise per grouping using training data.
